# EUROPEAN PATENT SPECIFICATION

(11) **EP 2 588 176 B1**
(45) Date of publication and mention of the grant of the patent: **24.08.2016**
(21) Application number: 11752339.9
(22) Date of filing: 28.06.2011
(51) Int. Cl.: A61M 16/00, A61M 16/06

(54) **SYSTEM FOR PERFORMING RESPIRATORY DIAGNOSTICS**
SYSTEM ZUR DURCHFÜHRUNG VON ATEMDIAGNOSEN
SYSTÈME DE RÉALISATION DE DIAGNOSTICS RESPIRATOIRES

(30) Priority: 02.07.2010 US 361037 P
(43) Date of publication of application: 08.05.2013
(73) Proprietor: Koninklijke Philips N.V., 5656 AE Eindhoven (NL)
(72) Inventor: COLBAUGH, Michael, Edward, NL-5656 AE Eindhoven (NL); WITT, Erik, Kurt, NL-5656 AE Eindhoven (NL)
(74) Representative: van Velzen, Maaike Mathilde
(86) International application number: PCT/IB2011/052832
(87) International publication number: WO 2012/001621

(56) References cited:
- WO-A1-2008/028248
- US-A- 5 345 930
- US-A1- 2004 237 965
- US-A1- 2007 017 522

## Description

The present invention relates to the performance of respiratory diagnostic testing to diagnose and/or develop treatment for respiratory disorders, such as obstructive sleep apnea, and in particular the a system for performing respiratory diagnostics that can deliver very low pressures to a subject using a standard a CPAP machine (as the source of pressure) and/or standard CPAP pressure and flow levels and/or passively with the use of dynamic inspiratory and expiratory resistance.

Diagnostic testing for a number of respiratory disorders requires the delivery of a flow of pressurized gas to the airway of the subject in order to observe the reaction of the subject thereto. In some diagnostic situations, it is necessary to deliver such a flow at very low pressures, and in particular at pressure levels that are below what a typical continuous positive airway pressure (CPAP) machine can deliver (as is known in the art, typical CPAP machines are only designed to (and to some degree capable of) providing gas pressures above about 4 cmH₂O). For example, in diagnosing and developing and/or monitoring/modifying a treatment regimen for obstructive sleep apnea, the critical PAP pressure at which a patient's airway will become more resistive and/or collapse must be determined, and such a determination requires the delivery of very low pressure levels. As a result, for this purpose, a special CPAP system is currently used that is referred to in some circles as a P_crit system.

A P_crit system is a fairly expensive and rare apparatus that is designed with multiple blower-compressors plus special sensors and valves to enable the device to smoothly transition the pressure in a respiratory mask from a higher CPAP pressure (typically at or above a patient's prescription pressure) to zero pressure. Typically, in a P_crit system, one blower is a positive pressure source, while the other is a negative pressure source. The control system in the P_crit system either blends the pressure from each blower, or with the use of a set of proportional valves, regulates the output pressure to some value between zero and a maximum CPAP pressure. More specifically, the P_crit system uses the addition of a negative pressure generator along with a positive pressure generator to precisely control the patient pressure all the way down to 0 cmH₂O (and negative if needed) for assessment of the critical pressure at which the upper airway collapses. P_crit systems are, however, fairly expensive and rare.

WO 2008/028248 A1 discloses a method and an apparatus for treating sleep-disordered breathing. Said apparatus may temporarily boost the pressure of a supply of breathable gas provided by an AutoSet device for the treatment of hypopnea. The presence and/or absence of apneas and/or hypopneas are detected by monitoring the Apnea-Hypopnea Index. When hypopnea events are detected, the pressure of the supply of breathable gas temporarily is increased above the Pcrit and/or CPAP levels, at least during patient inspiration. When the hypopnea events are normalized, the pressure is reduced.

US 5,345,930 A discloses an apparatus for assisting expulsional movement of pulmonary secretions via supramaximal flows. The device has a valve that opens fully in not more than 10 milliseconds, a pressure sensing means generating a signal indicative of a pressure in the upper part of the subject's respiratory tract, detecting means indicating the imminent collapse of the subject's respiratory tract or a part thereof, and control means opening or closing the valve respectively in response to the signals from the sensing means and the detecting means so as to create supramaximal flows in the respiratory tract in order to stimulate the movement of secretions towards the mouth of the subject.

There is thus a need for a system and method for performing respiratory diagnostics that can deliver very low pressures (including negative inspiratory pressures) to a subject using standard CPAP pressure and flow levels.

In one embodiment, a system for performing respiratory diagnostics is provided that includes a pressure generator configured to generate a first pressurized flow of breathable gas having a first pressure level above atmospheric pressure (e.g., at levels typical of common CPAP devices) and a patient interface device coupled to the pressure generator. The patient interface device includes a body having one or more subject interface openings and one or more valves disposed in the body, at least one of the one or more valves being selectively configurable to cause a second pressurized flow of breathable gas to be delivered through the one or more subject interface openings at a selectable pressure level anywhere between the first pressure level and atmospheric pressure in response to the patient interface receiving the first pressurized flow of breathable gas and without receiving any other pressurized flows of breathable gas.

These and other objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention. As used in the specification and in the claims, the singular form of "a", "an", and "the" include plural referents unless the context clearly dictates otherwise.
FIG. 1 is a schematic diagram of respiratory diagnostic system 2 according to one exemplary embodiment of the invention;
FIG. 2 is a pressure flow model of the respiratory diagnostic system of FIG. 1; and
FIGS. 3, 4, 5 and 6 are schematic diagrams of respiratory diagnostic systems according to alternative exemplary embodiments of the invention.

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

As employed, herein, the statement that two or more parts or components are "coupled" together shall mean that the parts are joined or operate together either directly or through one or more intermediate parts or components.

As employed herein, the statement that two or more parts or components "engage" one another shall mean that the parts exert a force against one another either directly or through one or more intermediate parts or components.

As employed herein, the term "number" shall mean one or an integer greater than one (i.e., a plurality).

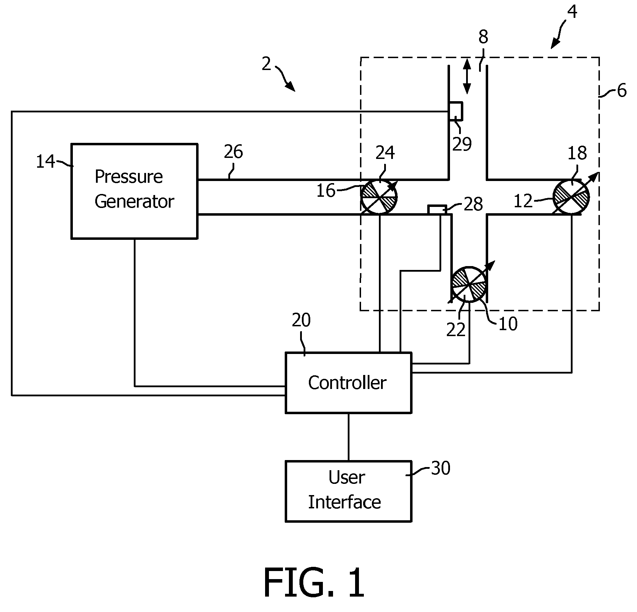

FIG. 1 is a schematic diagram of respiratory diagnostic system 2 according to one exemplary embodiment of the invention. As seen in FIG. 1, respiratory diagnostic system 2 includes variable resistance patient interface device 4. As described in greater detail herein, respiratory diagnostic system 2 and variable resistance patient interface device 4 forming a part thereof may be used to perform various respiratory diagnostic procedures on a subject that require very low pressures (e.g., from about 4 cmH₂O down to atmospheric pressure (0 cmH₂O)) using gas input pressures that are typical of known continuous positive airway pressure (CPAP) machines. As is known in the art, typical CPAP machines are only designed to (and to some degree capable of) provide gas pressures above about 4 cmH₂O. As described in greater detail herein, variable resistance patient interface device 4 is, with gas inputs at levels typical to known CPAP machines (e.g., about 4 cmH₂O and up), structured and configured to permit the pressure of the gas that is ultimately delivered to the airway of the subject to be selectively controlled to very low pressures (e.g. from about 4 cmH₂O to 0 cmH₂O).

Variable resistance patient interface device 4 includes body portion 6 that is structured to cover/enclose one or more external orifices of the airway of a subject. The body portion 6 may comprise, without limitation, a nasal mask that covers the subject's nose, a nasal pillow/cushion having nasal prongs that are received within the subject's nares, a nasal/oral mask that covers the subject's nose and mouth, or a full face mask that covers the subject's face. In the exemplary embodiment, variable resistance patient interface device 4 also includes a headgear component (not shown) which may be used to attach variable resistance patient interface device 4 to the subject's head.

Body portion 6 forms a plurality of openings with flow paths therebetween. In the exemplary embodiment, the interior of body portion 6 is hollow and without substantial impediment to the flow of gas from any of the various openings to any of the other various openings. The plurality of openings include one or more subject interface openings 8 (e.g., a pair of nasal prongs) for communicating gases from within body portion 6 to the airway of the subject, one or more inhalation ports 10 and one or more exhalation ports 12. Body portion 6 forms a first subset of flow paths between inhalation ports 10 and subject interface openings 8 that deliver gas from ambient atmosphere to one or more external orifices of the airway of the subject during inhalation. Body portion 6 also forms a second subset of flow paths between exhalation ports 12 and subject interface openings 8 that deliver gas from the one or more external orifices of the subject to ambient atmosphere during exhalation. In addition, in order to receive a pressurized flow of breathable gas from pressure generator 14, described in more detail below, body portion 6 includes circuit port 16 through which the pressurized gas is communicated to the interior of body portion 6.

Furthermore, in the illustrated embodiment, at the one or more exhalation ports 12, variable resistance patient interface device 4 includes one or more variable resistance exhalation valves 18. Variable resistance exhalation valves 18 permit gas to flow from body portion 6 to ambient atmosphere through the one or more exhalation ports 12, but significantly resist or seal the flow of gas from ambient atmosphere to body portion 6 through the one or more exhalation ports 12. For example, variable resistance exhalation valves 18 may be "one way" valves that permit gas to flow out of body portion 6 to atmosphere, but block gas from flowing into body portion 6 from atmosphere. The one or more variable resistance exhalation valves 18 may be dynamically controlled throughout the respiratory cycle to affect a "one way" operation characteristic (i.e. closed during inhalation, and returned to some lower resistance during exhalation). In addition, the resistance of variable resistance exhalation valves 18 to the flow of gas from the subject to ambient atmosphere is configurable to adjust the cumulative resistance of variable resistance patient interface device 4 to gas flowing through body portion 6 from subject interface openings 8 to atmosphere during exhalation. More specifically, in the illustrated embodiment, variable resistance exhalation valves 18 are operatively coupled to controller 20 forming a part of respiratory diagnostic system 2, and controller 20 is adapted to manipulate variable resistance exhalation valves 18 to selectively configure the resistance of variable resistance exhalation valves 18. The one or more variable resistance exhalation valves 18 may also be a variable or fixed resistance 2-way valve which may be set to a value which sets a fixed rate of flow resistance, providing a predetermined baseline rate of leak flow into and out of the mask (variable resistance patient interface device 4) during the full respiratory cycle. As discussed below, if the one or more variable resistance inhalation valves 22 shown in FIG. 1 is a 2-way variable resistance dynamically adjusted by controller 20, then various levels of respiratory resistance may be achieved that ranges from the value set by the one or more variable resistance exhalation valves 18 and downwards towards zero (0) resistance to flow into and out of the chamber that includes the one or more subject interface openings 8.

In addition, in the illustrated embodiment, at the one or more inhalation ports 10, variable resistance patient interface device 4 includes one or more variable resistance inhalation valves 22. Variable resistance inhalation valves 22 permit gas to flow from ambient atmosphere into the flow paths formed within body portion 6 through the one or more inhalation ports 10, but significantly resist or seal the flow of gas from within body portion 6 to ambient atmosphere through the one or more inhalation ports 10. For example, inhalation valves 22 may be "one way" valves that permit gas to flow into body portion 6 from atmosphere, but block gas within body portion 6 from flowing to atmosphere. In addition, the resistance of variable resistance inhalation valves 22 to the flow of gas from ambient atmosphere to the subject is configurable to adjust the cumulative resistance of variable resistance patient interface device 4 to gas flowing through body portion 6 from atmosphere to subject interface openings 8 during inhalation. More specifically, in the illustrated embodiment, variable resistance atmosphere valves 22 are operatively coupled to controller 20, and controller 20 is adapted to manipulate variable resistance inhalation valves 22 to selectively configure the resistance of variable resistance inhalation valves 22. As described in greater detail herein, the valves 18 and 22 may both be 2-way valves which are controlled by controller 20 and in conjunction with pressure generator 14 can produce practically any desired pressure in the central chamber of the interface connected to the patient through the one or more subject interface openings 8, from zero (0) to the full pressure capable in the pressure generator 14. In addition, the flow resistance of the valves 18 and 22 may be controlled, in cooperative control of the pressure generator 14 (described below), so that negative pressures may be produced during inhalation. This variant of control can produce an effective average pressure in the device below atmospheric pressure which can be used to further challenge the respiratory function beyond the atmospheric pressure setting of variable resistance patient interface device 4.

As seen in FIG. 1, in the illustrated embodiment, variable resistance circuit valve 24 is disposed within circuit port 16 to control the flow rate of the pressurized flow of breathable gas that enters body portion 6 through circuit port 16. Limiting flow from/to the pressure generator 14 enables diagnosis of the airway utilizing the variable pressure characteristics developed by the patient's inspiration and exhalation and the total net respiration resistance of variable resistance patient interface device 4. In this pressure generator flow limitation case, the dynamic control of valve(s) 22 and/or valve(s) 18 is enabled to determine more of the pressure characteristics that the patient will experience.

As noted above, respiratory diagnostic system 2 includes pressure generator 14. Pressure generator 14 is coupled to variable resistance patient interface device 4 through circuit 26. Pressure generator 14 is configured to generate a pressurized flow of breathable gas for delivery to the airway of the subject via variable resistance patient interface device 4. As seen in FIG. 1, pressure generator 14 is coupled to and under the control of controller 20. Controller 20 includes a processing portion which may be, for example, a microprocessor, a microcontroller or some other suitable processing device, and a memory portion that may be internal to the processing portion or operatively coupled to the processing portion and that provides a storage medium for data and software executable by the processing portion for controlling the operation of respiratory diagnostic system 2 as described herein.

One or more parameters of the pressurized flow of breathable gas generated by pressure generator 14 may, under the control of controller 20, be controlled by pressure generator 14 for diagnostic purposes as described elsewhere herein. For example, pressure generator 14 may control one or more of the pressure, the flow rate, the composition, and/or other parameters of the pressurized flow of breathable gas. In the exemplary embodiment, pressure generator 14 includes a gas source and one or more components that control the flow and/or pressure of a pressurized flow of gas generated from the gas within the gas source. The gas source may include any supply (or supplies) of breathing gas, such as, for example, ambient atmosphere, a tank of pressurized gas, a wall gas source, and/or other bodies of breathable gas. The breathing gas from the gas source can be any breathable gas, such as air, oxygen, an oxygen mixture, a mixture of a breathing gas and a medication, which can be in gaseous form (*e.g*., nitric oxide, nebulized, *etc*.), and/or other breathable gases. The one or more components that control one or more parameters of the pressurized flow of breathable gas may include one or more of a valve, a blower, a piston, a bellows, and/or other mechanisms for controlling one or more parameters of the pressurized flow of breathable gas.

Circuit 26 forms a gas flow path between pressure generator 14 and variable resistance patient interface device 4. The gas flow path formed by circuit 26 delivers the pressurized flow of breathable gas generated by pressure generator 14 from pressure generator 14 to variable resistance patient interface device 4. In the exemplary embodiment, circuit 26 includes a flexible conduit that runs between pressure generator 14 and variable resistance patient interface device 4 and that substantially seals the flow path between pressure generator 14 and variable resistance patient interface device 4 from atmosphere.

As seen in FIG. 1, respiratory diagnostic system 2 also includes one or more sensors 28 that are coupled to controller 20 and are configured to generate one or more output signals that convey information relating to the stability of the airway of the subject that is wearing variable resistance patient interface device 4. In the illustrated embodiment, the one or more sensors 28 are carried on body portion 6. Alternatively, the one or more sensors 28 may be carried by circuit 26. By way of non-limiting example, the one or more sensors 28 may monitor one or more gas parameters at or near the airway of the subject (e.g., within body portion 6). The one or more parameters may include one or more of flow, pressure, and/or other parameters. In one particular, non-limiting embodiment, the one or more sensors 28 include a transducer that converts vibration to an electrical output signal. The output signal generated by the transducer may convert vibration in sound waves generated by instability in the airway of the subject (e.g., "snoring"), vibration of tissues around the airway of the subject caused by airway instability, and/or other vibrations indicative of airway instability. Sensor 29 may also be included (on or within body 6), which monitors flow and/or other parameters that are specific to the path fluidly connected to the patient through the one or more subject interface openings 8. It should be understood that sensors 28 and 29 represent a variety of sensors which may be applied in a way to provide specific flows or other parameters which are specific to any and all ports of variable resistance patient interface device 4 (e.g. ports 8, 12, 10, and 16).

Finally, respiratory diagnostic system 2 includes user interface 30 that is configured to provide an interface between respiratory diagnostic system 2 and a user (e.g., a caregiver or other health professional) to enable information, such as control information, to be input into respiratory diagnostic system 2 and to enable information, such as diagnostic information or results, to be output by respiratory diagnostic system 2. Other sensors, monitoring systems, and/or diagnostic systems applied to the patient or test environment may also tie into the respiratory diagnostic system 2 of the present invention, to alter the control of the system or to be recorded along with other parameters generated by respiratory diagnostic system 2. Likewise parameters generated by respiratory diagnostic system 2 may also be output to other systems associated with the patient and/or test environment.

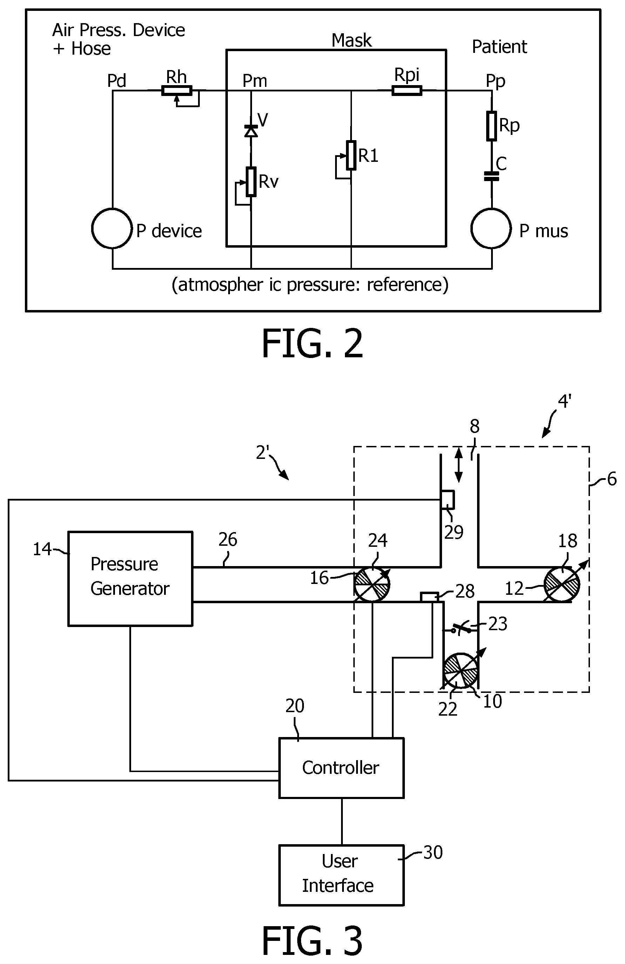

FIG. 2 is a pressure flow model of respiratory diagnostic system 2 which, along with FIG. 1, will be used to explain an exemplary embodiment of the present invention. In FIG. 2, "P device" represents pressure generator 14, "Mask" represents variable resistance patient interface device 4, "Pd" represents the pressure of the gas generated by pressure generator 14, "Rh" represents circuit valve 24 and its associated resistance, "Rv" represents the one or more inhalation valves 22 and their associated resistance, "Rl" represents the one or more exhalation valves 18 and their associated resistance, "Pm" represents the pressure within variable resistance patient interface device 4, and "Pp" represents the pressure delivered to the subject (the "Patient").

As noted elsewhere herein, in the present invention, the pressure of the air circuit can be varied to very low pressures (even as low as atmospheric pressure) using a standard CPAP machine (as the pressure source) and/or standard CPAP pressure and flow levels (generated by pressure generator 14) by controlling and/or adjusting the resistances of the ports of variable resistance patient interface device 4. In other words, with reference to FIG. 2, Rv, Rl and Rh can be controlled (selected and/or adjusted) to adjust the control of Pm (i.e., the pressure in variable resistance patient interface device 4) smoothly from a high value (e.g., 20 cmH₂O) all the way to zero (atmospheric) pressure, and even a negative pressure during inhalation. More specifically, in the exemplary embodiment illustrated in FIG. 1, by selecting and/or adjusting the resistances of the one or more exhalation valves 18, the one or more inhalation valves 22, and circuit valve 24, the pressure in variable resistance patient interface device 4 can be smoothly adjusted from a high value (e.g., 10-20 cmH₂O or higher) all the way to zero (atmospheric) pressure, and even to a negative pressure during inhalation.

For example, and without limitation, the one or more inhalation valves 22 and their associated resistance Rv can be controlled, with Rh and Rl each set to a selected high resistance, to adjust the amount of inhalation air flow in order to draw a negative pressure in variable resistance patient interface device 4 during inspiration. As described elsewhere herein, all of these factors can be controlled locally to variable resistance patient interface device 4. Thus, even with a relatively simple CPAP device being used as pressure generator 14 that controls Pd to a constant pressure, Rh Rv and Rl can be selected and/or varied to regulate Pm to a desired pressure that can be smoothly controlled down to zero. In one particular, non-limiting example, this may be done in the manner described below. For simplicity, the following description will treat the inspiratory and expiratory phases separately, and all resistances will be identified in units of 1 unit = cmH₂O/LPS measured at 0.1 LPS flow rate. Of course, the pressure characteristic of a fixed resistance varies by the square of the flow applied, so this single value only defines one point in the pressure/flow curve, but all values of a curve for a resistor of 2 units will, for all flows, produce twice the pressure of a resistor of 1 unit. Intentional leak port Rl is set to a reasonable but relatively high fixed value of 7 units (i.e. at the high end of the range of commercially available CPAP masks). A standard CPAP device produces regulated pressures at its output port down to 4 cmH₂O and beyond 25 cmH₂O. A typical sleeping patient peak expiratory flow is 0.2 LPS and a peak inspiratory flow is 0.4 LPS (note: the expiratory time is approximately twice that of inspiratory time). A typical patient circuit 26 has a resistance on the order of 0.01 units. Rh will be taken to vary from 0.01 units to being completely closed (i.e. flow is zero, for the defined pressures), and Rv will be taken to vary from 0.1 units to being completely closed. For PAP set-points of 4 cmH₂O or greater, with Rh=0.01, Rv=closed, an available CPAP or ventilatory pressure generator can provide adequate dynamic pressure control to meet the patient's flow demand over the whole respiratory cycle. At approximately 2-4 cmH₂O the common devices are not able to adequately regulate the pressure to the setting because during exhalation the blowers would have to slow to a stall in order to match the negative flow yet try to keep regulating pressure to the setting. For any dynamic pressure regulation set-point between 0 and 4 cmH₂O, the pressure generator would be programmed to stop decreasing its head pressure below a minimum pressure (e.g. 2 cmH₂O) during the exhalation phase, and Rh would be increased while Rv is decreased to keep the pressure at port 8 relatively constant at the set-point. For example, with values described above, Rl=7 units, with a set-point of 0 cmH₂O, at the end of inhalation, as the patient's flow slows, and throughout exhalation, the pressure would not drop below 2 cmH₂O unless Rh and Rv are changed. When Pd drops to the 2 cmH₂O minimum, and the patient inspiratory flow continues to decrease, Rh can be increased gradually to the value of Rl for instance (i.e. 7 units), matching inspiratory flow. As inspiratory flow continues to decrease, Rv must be decreased to provide the extra flow to keep Pm at 0 cmH₂O. As respiration transitions to the expiratory phase Rv is decreased further to its minimum, and Rh increased further to its maximum. With the dynamic control ranges for the resistors described above, Rh=closed, and Rv=0.1 units, at peak expiration, Pm would be 0.2 LPS * 4 * 0.0986 units = 0.08 cm (note: the 4 factor is because the flow is 2 times 0.1 LPS, and the resistance is Rv=0.1 in parallel with Rl=7). At peak exhalation the control system can keep the pressure from exceeding 0.1 cmH20; essentially zero for these tests. This is a method for achieving constant pressure control with realistic devices, from the pressure generator's maximum setting to a zero set-point, where the pressure generator's pressure never drops below 2 cmH₂O. Of course, the same devices may be used in this way for generators with a minimum pressure greater than 2 cmH₂O as well.

Thus, because the pressure Pm within variable resistance patient interface device 4 can be smoothly adjusted from a high value (e.g., 10-20 cmH₂O or higher) all the way down to zero (atmospheric) pressure, respiratory diagnostic system 2 may be used to perform diagnostic testing for respiratory disorders in situations that require the delivery of a flow of pressurized gas to the airway of the subject at very low pressures, and this may be done using a standard CPAP machine and/or standard CPAP pressure and flow levels.

In one exemplary, non limiting embodiment, the present invention may be used to determine the critical PAP pressure at which a patient's airway will become more resistive and/or collapse, which, as described elsewhere herein is important for diagnosis and treatment of obstructive sleep apnea. More specifically, respiratory diagnostic system 2 may determine the critical PAP pressure of a subject in the following manner. First, pressure is delivered to the subject using respiratory diagnostic system 2 at the holding/prescription pressure of the subject (which is the pressure at which it is known that the subject will not experience an apnea event) for a certain period of time (e.g., 1-10 minutes or 5-150 breaths). Then, the pressure delivered by respiratory diagnostic system 2 is adjusted downwardly (as described elsewhere herein) by an incremental amount (e.g., 1 cmH₂O) to a first challenge pressure for a certain number (e.g., five) of breaths, after which time the pressure is returned to the holding pressure. During the time at which the pressure is at the first challenge pressure, information from the one or more sensors 28 and/or 29 is used to determine whether the first challenge pressure caused the airway of the subject to collapse. If the airway is determined to have collapsed due to the first challenge pressure, then the first chilling pressure may be determined to be the critical PAP pressure. If, however, the airway is determined to not have collapsed due to the first challenge pressure, then the process is repeated a number of times, with the challenge pressure dropping successively and incrementally each time (possibly to the very low pressures described herein), until a challenge pressure is reached at which the airway is determined to have collapsed. Again, due to the features of variable resistance patient interface device 4 described elsewhere herein, this (the pressure variation) may all be done using standard CPAP pressure and flow levels that are delivered to variable resistance patient interface device 4.

It should be understood that the above description is meant to be exemplary only that that the present invention is not limited to diagnostic testing for determining the critical PAP pressure at which a patient's airway will become more resistive and/or collapse. Rather, the present invention may be used for performing other diagnostic measures including, without limitation, measuring lung compliance, measuring airway resistance, and diagnosing conditions like acute pulmonary edema, COPD, sleep disordered breathing and upper airway instability, including snoring.

Furthermore, the present invention also enables the ability to alter the exhalation resistance to test the reaction of the subject's body thereto for diagnostic purposes. As described elsewhere herein, variable resistance patient interface device 4 can be designed to dynamically vary the exhalation resistance, and/or the inhalation resistance, as well as maintain a desired level of pressure anytime throughout the respiration cycle.

Because the present invention concerns controlling or adjusting resistance of the ports of a mask such as variable resistance patient interface device 4, the inclusion of a local fresh air source therein (inhalation valve 22), along with the use of sensing and control of airflow into/out of the mask (common CPAP), it enables more complete and fine control of mask pressure, breathing resistance, and CO₂ retention in the mask. Without a valve such as inhalation valve 22, and with fixed Rh and Rl, a common P_crit system has a disadvantage of building retained CO₂ in its hose when the pressure source P_device is near or below atmospheric pressure. The inhalation valve of the present invention can be set to supply fresh air upon inhalation, Rl can be reduced to enable more flow of exhaled gas out of the mask, plus the resistance Rh can be made higher than in common systems. All or any of this additional control (i.e. beyond control of Pd alone) results in better minimization or control of the level of CO₂ in the mask.

FIG. 3 is a schematic diagram of respiratory diagnostic system 2' according to an alternative exemplary embodiment of the invention. Respiratory diagnostic system 2' includes a number of the same components as respiratory diagnostic system 2, and like components are labeled with like reference numerals. In addition, respiratory diagnostic system 2' may also be modeled by the pressure flow model of FIG. 2, and operation of respiratory diagnostic system 2' (i.e., the control of pressure) is similar to operation of respiratory diagnostic system 2 described above. However, as seen in FIG. 3, in respiratory diagnostic system 2', the resistances of exhalation valve 18 and inhalation valve 22, rather than being automatically controlled by controller 20, are manually controllable. In this illustrative embodiment, only circuit valve 24 is dynamically controlled by controller 20 to work in conjunction with pressure generator 14 to provide pressure and circuit flow control. In this alternative embodiment, either port/path 12 or 10, or both, must include a mechanical "one way" valve to separate the characteristics of flow resistance during the inspiratory and expiratory phase of the respiratory cycle. In FIG. 3, one-way valve 23 is illustrated to force expiratory flow to only occur through valves 18 and/or 24. Furthermore, for example, one or more of the valves just mentioned may have manually adjustable components that allow the resistance thereof to be selectively adjusted. Alternatively, one or more of the valves just mentioned may include fixed resistance valves that are selectively detachable from the associated ports for replacement by valves having the desired resistance. The adjustment of resistance of the valves just mentioned may include an adjustment to a diameter, a cross-sectional size, and/or an area of one or more openings in body portion 6 associated with the valve.

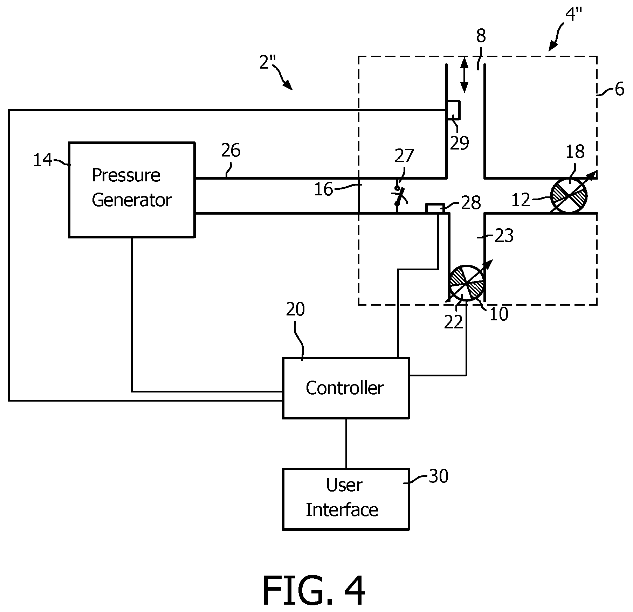

FIG. 4 is a schematic diagram of respiratory diagnostic system 2" according to still a further alternative exemplary embodiment of the invention. Respiratory diagnostic system 2" includes a number of the same components as respiratory diagnostic system 2, and like components are labeled with like reference numerals. In addition, respiratory diagnostic system 2" may also be modeled by the pressure flow model of FIG. 2, and operation of respiratory diagnostic system 2" (i.e., the control of pressure) is similar to operation of respiratory diagnostic system 2 described above. However, as seen in FIG. 4, in respiratory diagnostic system 2", while the resistance of exhalation valve 18 in variable resistance patient interface device 4" is set at a desired resistance value, the resistance of inhalation valve 22 in variable resistance patient interface device 4" is automatically controlled by controller 20, and the resistance at circuit port 16 is fixed and determined by the properties (e.g., diameter) of circuit 26. Thus, a particular circuit 26 will be chosen to achieve a desired resistance in order to otherwise enable variable resistance patient interface device 4" to adjust pressure as described herein based on the resistances of the one or more exhalation valves 18 and/or the one or more inhalation valves 22. A one-way valve 27 may be used in the path of port 16 to minimize the amount of exhaust gas that can travel down circuit 26.

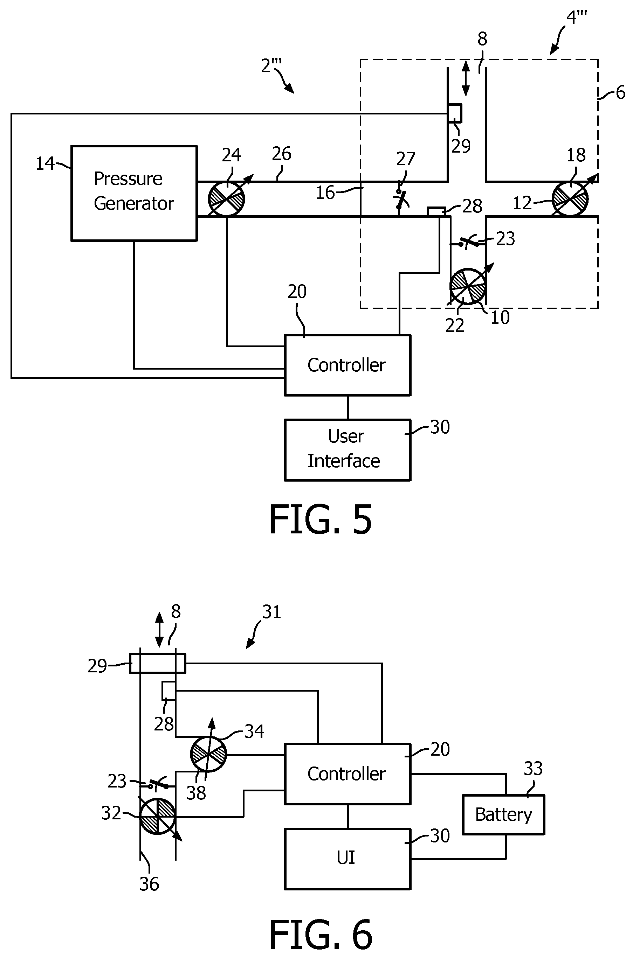

FIG. 5 is a schematic diagram of respiratory diagnostic system 2"' according to an alternative exemplary embodiment of the invention. Respiratory diagnostic system 2"' is similar to respiratory diagnostic system 2", except that the resistances of the one or more exhalation valves 18 and the one or more inhalation valves 22 are manually controllable as described elsewhere herein. Note that valve 27 may be employed as described elsewhere in the path of port 16, and valve 23 is a one-way valve which functions to enable the inspiratory resistance (valve 18 in parallel with valve 22) from being different than the expiratory resistance (valve 18). Of course, an additional valve like valve 23 may be employed in the path of port 12, but designed to allow only expiratory flow; enabling the expiratory resistance to be set by valve 18 alone, and inspiratory resistance to be set by valve 22 alone. Valve 24 is moved to the pressure generator 14 end of circuit 26 or within the pressure generator 14 body to enable flow into circuit 26 to be restricted while the pressure generator 14 is producing a non-zero head pressure. Note that this configuration has only sensory connections from variable resistance patient interface device 4"' and that all components operate passively without control drive. This configuration tends to be lower cost and more compact than all of the other embodiments described to this point.

An even more minimal embodiment of the invention is illustrated in FIG. 6, which does not include a pressure generator at all and which enables the development/regulation of positive expiratory pressure, but also provides separate control for development/regulation of atmospheric-to-negative range inspiratory pressure as well. In this configuration, patient interface device 31 is provided that includes controller 20 that is coupled with dynamically controlled, variable resistance valve 32 provided in port 36 and dynamically controlled, variable resistance valve 34 provided in port 38. One-way valve 23 is included to set-up valve 34 as the sole resistance for exhalation, and enables the inspiratory resistance to be set separately to a lower value with valve 32. As described elsewhere, controller 20 takes in information from sensors 28 and 29, and provides actuation of valves 32 and 34, to develop the desired flow control for the diagnostic program. This variant is not able to produce inspiratory pressures greater than zero (0) nor expiratory pressures greater than a value at which the patient can comfortably breath, but it is implemented in a very simple configuration with very low power required to operate and does not need the patient circuit 26, which enables a free range of motion. The embodiment of FIG. 6 can be implemented as a small self-contained battery-operated mask wherein power is provided by battery 33. The user interface 30 may be remotely connected functionally via wireless link to controller 20, or may be wired.

Since the alternative embodiments described herein allow the pressure delivered to the subject to be smoothly adjusted from a high value (e.g., 10-20 cmH₂O or higher) all the way down to zero (atmospheric) pressure, those embodiments, like respiratory diagnostic system 2, may also be used to perform diagnostic testing for respiratory disorders (for example as described herein) in situations that require the delivery of a flow of pressurized gas to the airway of the at very low pressures, and this may be done using a standard CPAP machine and/or standard CPAP pressure and flow levels.

Moreover, it should be understood that the embodiments shown in FIGS. 1, 3, 4, 5 and 6 are meant to be exemplary only, and that other variations are possible within the scope of the present invention. For example, combinations of the various embodiments are possible wherein some valves are automatically controllable as described elsewhere herein while other valves are manually controllable as also described elsewhere herein. Also, one or more of the valves maybe fixed and non-removable in nature. As another example, Unites States Patent Application Nos. 61/141,250; 61/141,251; and 61/141,252, assigned to the assignee of the present invention, each describe one or more embodiments of a patient interface device that is similar to variable resistance patient interface device 4, and those embodiments may also be used to implement the present invention as described herein (e.g., as a substitute for variable resistance patient interface device 40.

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A system (2, 2', 2", 2"') for performing respiratory diagnostics, comprising:
a pressure generator (14) configured to generate a first pressurized flow of breathable gas having a first pressure level above atmospheric pressure; and
a patient interface device (4, 4', 4", 4"') coupled to the pressure generator, the patient interface device including a body (6) having one or more subject interface openings (8)
**characterized in that** the patient interface device (4, 4', 4", 4"') further includes one or more valves (18, 22, 24) disposed in the body (6), at least one of the one or more valves (18, 22, 24) being selectively configurable to cause a second pressurized flow of breathable gas to be delivered through the one or more subject interface openings (8) at a selectable pressure level anywhere between the first pressure level and atmospheric pressure in response to the patient interface receiving the first pressurized flow of breathable gas and without receiving any other pressurized flows of breathable gas.

2. The system according to claim 1, wherein the body (6) has one or more inhalation ports (10) and the one or more valves (18, 22, 24) include one or more inhalation valves (22) provided at the one or more inhalation ports, wherein the one or more inhalation valves (22) have a first resistance to gas flow into the body (6) from atmosphere that is selectively adjustable, wherein a value of the first resistance determines the selectable pressure level.

3. The system according to claim 2, wherein the body (6) has one or more exhalation ports (12) and the one or more valves (18, 22, 24) include one or more exhalation valves (18) provided at the one or more exhalation ports, wherein the one or more exhalation valves (18) have a second resistance to gas flow out of the body (6) to atmosphere.

4. The system according to claim 3, wherein the second resistance is selectively adjustable.

5. The system according to claim 3, wherein the body (6) has a circuit port (16) structured to be coupled to the pressure generator (14) through a circuit (26).

6. The system according to claim 5, wherein the one or more valves (18, 22, 24) include a circuit valve (24) provided at the circuit port (16) having a third resistance to gas flow into the body (6) through the circuit port (16).

7. The system according to claim 6, wherein the third resistance is selectively adjustable.

8. The system according to claim 2, further comprising a controller (20) coupled to the pressure generator and the one or more inhalation valves, the controller being adapted to control the first pressure level and to automatically adjust the first resistance.

9. The system according to claim 4, further comprising a controller coupled to the pressure generator (14), the one or more inhalation valves (22), and the one or more exhalation valves (18), the controller being adapted to control the first pressure level and to automatically adjust the first resistance and the second resistance.

10. The system according to claim 1, further comprising a controller (20) and one or more sensors (28) coupled to the controller (20) and configured to generate one or more output signals that convey information related to a stability of an airway of a subject wearing the patient interface device (4, 4', 4", 4"').

11. The system according to claim 10, wherein controller (20) is coupled to the pressure generator (14) and the one or more valves (18, 22, 24), wherein the controller (20) is adapted to configure the one or more valves (18, 22, 24), control the selectable pressure level and generate diagnostic information based on the information related to the stability of the airway of the subject provided to the controller (20) in response to the second pressurized flow of breathable gas being delivered to the subject.

12. The system according to claim 11, wherein the diagnostic information is a critical PAP pressure for the subject.

## Patentansprüche

1. System (2, 2', 2", 2"') zur Durchführung von Atemdiagnosen, das Folgendes umfasst:
einen Druckgenerator (14), der konfiguriert ist, um eine erste unter Druck stehende Atemgasströmung mit einem ersten Druckniveau über dem atmosphärischen Druck zu erzeugen; und
eine Patienten-Schnittstellenvorrichtung (4, 4', 4", 4"'), die mit dem Druckgenerator gekoppelt ist, wobei die Patienten-Schnittstellenvorrichtung einen Körper (6) mit einer oder mehreren Personen-Schnittstellenöffnungen (8) umfasst,
**dadurch gekennzeichnet, dass** die Patienten-Schnittstellenvorrichtung (4, 4', 4", 4"') weiterhin ein oder mehrere in dem Körper (6) angeordnete Ventile (18, 22, 24) umfasst, wobei mindestens eines der einen oder mehreren Ventile (18, 22, 24) selektiv konfigurierbar ist, um zu bewirken, dass in Reaktion darauf, dass die Patientenschnittstelle die erste unter Druck stehende Atemgasströmung empfängt, ohne irgendwelche anderen unter Druck stehenden Atemgasströme zu empfangen, eine zweite unter Druck stehende Atemgasströmung mit einem irgendwo zwischen dem ersten Druckniveau und dem atmosphärischen Druck auswählbaren Druckniveau durch die eine oder mehrere Personen-Schnittstellenöffnungen (8) abgegeben wird.

2. System nach Anspruch 1, wobei der Körper (6) einen oder mehrere Einatmungsanschlüsse (10) hat und das eine oder mehrere Ventile (18, 22, 24) ein oder mehrere Einatmungsventile (22) umfassen, die an dem einen oder mehreren Einatmungsanschlüssen vorgesehen sind, wobei das eine oder mehrere Einatmungsventile (22) einen ersten Widerstand gegen eine Gasströmung aus der Atmosphäre in den Körper (6) haben, der selektiv einstellbar ist, wobei ein Wert des ersten Widerstands das auswählbare Druckniveau bestimmt.

3. System nach Anspruch 2, wobei der Körper (6) einen oder mehrere Ausatmungsanschlüsse (12) hat und das eine oder mehrere Ventile (18, 22, 24) ein oder mehrere Ausatmungsventile (18) umfassen, die an dem einen oder mehreren Ausatmungsventilen vorgesehen sind, wobei das eine oder mehrere Ausatmungsventile (18) einen zweiten Widerstand gegen eine Gasströmung aus dem Körper (6) zur Atmosphäre haben.

4. System nach Anspruch 3, wobei der zweite Widerstand selektiv einstellbar ist.

5. System nach Anspruch 3, wobei der Körper (6) einen Kreislaufanschluss (16) hat, der konstruiert ist, um über einen Kreislauf (26) mit dem Druckgenerator (14) gekoppelt zu werden.

6. System nach Anspruch 5, wobei das eine oder mehrere Ventile (18, 22, 24) ein Kreislaufventil (24) umfassen, das an dem Kreislaufanschluss (16) vorgesehen ist und einen dritten Widerstand gegen eine Gasströmung durch den Kreislaufanschluss (16) in den Körper (6) aufweist.

7. System nach Anspruch 6, wobei der dritte Widerstand selektiv einstellbar ist.

8. System nach Anspruch 2, weiterhin umfassend eine Steuereinheit (20), die mit dem Druckgenerator und dem einen oder mehreren Einatmungsventilen gekoppelt ist, wobei die Steuereinheit dafür ausgelegt ist, das erste Druckniveau zu steuern und den ersten Widerstand automatisch einzustellen.

9. System nach Anspruch 4, weiterhin umfassend eine Steuereinheit, die mit dem Druckgenerator (14), dem einen oder mehreren Einatmungsventilen (22) und dem einen oder mehreren Ausatmungsventilen (18) gekoppelt ist, wobei die Steuereinheit dafür ausgelegt ist, das erste Druckniveau zu steuern und den ersten Widerstand sowie den zweiten Widerstand automatisch einzustellen.

10. System nach Anspruch 1, weiterhin umfassend eine Steuereinheit (20) und einen oder mehrere Sensoren (28), die mit der Steuereinheit (20) gekoppelt sind und konfiguriert sind, um ein oder mehrere Ausgangssignale zu erzeugen, die Informationen in Bezug auf eine Stabilität eines Atemwegs einer Person übermitteln, die die Patienten-Schnittstellenvorrichtung (4, 4', 4", 4"') trägt.

11. System nach Anspruch 10, wobei die Steuereinheit (20) mit dem Druckgenerator (14) und dem einen oder mehreren Ventilen (18, 22, 24) gekoppelt ist, wobei die Steuereinheit (20) dafür ausgelegt ist, das eine oder mehrere Ventile (18, 22, 24) zu konfigurieren, das auswählbare Druckniveau zu steuern und Diagnoseinformationen zu erzeugen, und zwar basierend auf den Informationen in Bezug auf die Stabilität des Atemwegs der Person, die der Steuereinheit (20) bereitgestellt werden, und in Reaktion darauf, dass die zweite unter Druck stehende Atemgasströmung an die Person abgegeben wird.

12. System nach Anspruch 11, wobei die Diagnoseinformation ein kritischer PAP-Druck für die Person ist.

## Revendications

1. Système (2, 2', 2", 2"') de réalisation de diagnostics respiratoires, comprenant :
un générateur de pression (14) configuré pour générer un premier flux de gaz respirable sous pression ayant un premier niveau de pression au-dessus de la pression atmosphérique ; et
un dispositif d'interface patient (4, 4', 4", 4"') couplé au générateur de pression, le dispositif d'interface patient comprenant un corps (6) ayant une ou plusieurs ouvertures d'interface sujet (8) ;
**caractérisé en ce que** le dispositif d'interface patient (4, 4', 4", 4"') comprend en outre une ou plusieurs valves (18, 22, 24) disposées dans le corps (6), au moins l'une de la/des valves (18, 22, 24) étant configurable de manière sélective pour amener un second flux de gaz respirable sous pression à être délivré à travers l'ouverture ou les ouvertures d'interface sujet (8) à un niveau de pression sélectionnable n'importe où entre le premier niveau de pression et la pression atmosphérique en réponse à la réception par l'interface patient du premier flux de gaz respirable sous pression et sans réception d'autres flux de gaz respirable sous pression.

2. Système selon la revendication 1, dans lequel le corps (6) possède un ou plusieurs orifices d'inhalation (10) et la/les valve(s) (18, 22, 24) comprend/comprennent une ou plusieurs valves d'inhalation (22) prévue(s) au niveau du/des orifice(s) d'inhalation, dans lequel la/les valve(s) d'inhalation (22) présente(nt) une première résistance au flux de gaz dans le corps (6) à partir de l'atmosphère qui peut être réglée de manière sélective, dans lequel une valeur de la première résistance détermine le niveau de pression sélectionnable.

3. Système selon la revendication 2, dans lequel le corps (6) possède un ou plusieurs orifices d'exhalation (12) et la/les valve(s) (18, 22, 24) comprend/comprennent une ou plusieurs valves d'exhalation (18) prévue(s) au niveau du/des orifice(s) d'exhalation, dans lequel la/les valve(s) d'exhalation (18) présente(nt) une seconde résistance au flux de gaz sortant du corps (6) vers l'atmosphère.

4. Système selon la revendication 3, dans lequel la seconde résistance est réglable de manière sélective.

5. Système selon la revendication 3, dans lequel le corps (6) possède un orifice de circuit (16) structuré pour être couplé au générateur de pression (14) par le biais d'un circuit (26).

6. Système selon la revendication 5, dans lequel la/les valve(s) (18, 22, 24) comprend/comprennent une valve de circuit (24) prévue au niveau de l'orifice de circuit (16) ayant une troisième résistance au flux de gaz dans le corps (6) à travers l'orifice de circuit (16).

7. Système selon la revendication 6, dans lequel la troisième résistance est réglable de manière sélective.

8. Système selon la revendication 2, comprenant en outre un régulateur (20) couplé au générateur de pression et à la/aux valve(s) d'inhalation, le régulateur étant conçu pour réguler le premier niveau de pression et pour régler automatiquement la première résistance.

9. Système selon la revendication 4, comprenant en outre un régulateur couplé au générateur de pression (14), à la/aux valve(s) d'inhalation (22) et à la/aux valve(s) d'exhalation (18), le régulateur étant conçu pour réguler le premier niveau de pression et pour régler automatiquement la première résistance et la seconde résistance.

10. Système selon la revendication 1, comprenant en outre un régulateur (20) et un ou plusieurs capteurs (28) couplé(s) au régulateur (20) et configuré(s) pour générer un ou plusieurs signaux de sortie qui acheminent des informations relatives à une stabilité de voies aériennes d'un sujet portant le dispositif d'interface patient (4, 4', 4", 4"').

11. Système selon la revendication 10, dans lequel le régulateur (20) est couplé au générateur de pression (14) et à la/aux valve(s) (18, 22, 24), dans lequel le régulateur (20) est conçu pour configurer la/les valve(s) (18, 22, 24), réguler le niveau de pression sélectionnable et générer des informations de diagnostic sur la base des informations relatives à la stabilité des voies aériennes du sujet fournie au régulateur (20) en réponse au second flux de gaz respirable sous pression délivré au sujet.

12. Système selon la revendication 11, dans lequel les informations de diagnostic sont une pression PAP critique pour le sujet.
